(19) Europäisches Patentamt
European Patent Office
Office européen des brevets

(11) **EP 3 536 162 B1**

(12) **EUROPEAN PATENT SPECIFICATION**

(45) Date of publication and mention
of the grant of the patent:
**21.12.2022 Bulletin 2022/51**

(21) Application number: **17867151.7**

(22) Date of filing: **18.10.2017**

(51) International Patent Classification (IPC):
***A23K 10/30*** (2016.01)      ***A23K 10/12*** (2016.01)
***A23K 50/75*** (2016.01)      ***A23K 50/90*** (2016.01)

(52) Cooperative Patent Classification (CPC):
**A23K 10/12; A23K 10/30; A23K 50/75;**
**A23K 50/90;** Y02A 40/818; Y02P 60/87

(86) International application number:
**PCT/JP2017/037681**

(87) International publication number:
**WO 2018/083983 (11.05.2018 Gazette 2018/19)**

(54) **FEED AND METHOD FOR MANUFACTURING SAME**

FUTTER UND VERFAHREN ZUR HERSTELLUNG DAVON

ALIMENT POUR ANIMAUX, ET PROCÉDÉ DE FABRICATION DE CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.11.2016  JP 2016215404**

(43) Date of publication of application:
**11.09.2019 Bulletin 2019/37**

(73) Proprietor: **Boskein Nutrition Limited
N.T., Hong Kong (CN)**

(72) Inventors:
• **KIKUSHIMA, Sunao**
**Kameoka-shi**
**Kyoto 621-0014 (JP)**
• **SHO, Sakiko**
**Kyoto-shi**
**Kyoto 606-0002 (JP)**
• **KANO, Yasuhiro**
**Kyoto-shi**
**Kyoto 601-1247 (JP)**

(74) Representative: **Krauns, Christian
Wallinger Ricker Schlotter Tostmann
Patent- und Rechtsanwälte Partnerschaft mbB
Zweibrückenstraße 5-7
80331 München (DE)**

(56) References cited:
**EP-A2- 0 243 826       WO-A2-2012/038509
CN-A- 105 285 626       JP-A- 2016 013 122
JP-A- 2016 088 918       JP-B2- 4 093 956**

• **BIGELIS R: "FUNGAL ENZYMES IN FOOD
PROCESSING", HANDBOOK OF APPLIED
MYCOLOGY, XX, XX, 1 January 1991 (1991-01-01),
pages 445-498, XP001051671,**
• **NORA PAP ET AL: "Effect of Ultrafiltration on
Anthocyanin and Flavonol Content of Black
Currant Juice (L.)", FOOD AND BIOPROCESS
TECHNOLOGY ; AN INTERNATIONAL JOURNAL,
SPRINGER-VERLAG, NEW YORK, vol. 5, no. 3, 15
May 2010 (2010-05-15), pages 921-928,
XP035025617, ISSN: 1935-5149, DOI:
10.1007/S11947-010-0371-Z**

**Description**

Technical Field

The present invention relates to a feed and a production method thereof and relates to a feed which can be widely used in the livestock industry, the poultry industry, the fish culture industry and which has a strong effect of increasing the body weight of the subject organism and a production method thereof.

Background Art

In the livestock industry for raising livestock such as cows, pigs, horses, sheep and camels or poultry such as chickens, ducks, crossbreeds between wild and domestic ducks, pheasants, quails, turkeys, guinea fowl, peafowl, geese, pigeons, ostriches and emus and in the fish culture industry for raising aquatic organisms such as fish and shellfish, it is required to further promote the growth of the subject organism to be raised and more efficiently increase the body weight of the subject organism as well as to prevent or treat diseases due to viral infection.

As preventive or therapeutic measures against the diseases, medicaments such as antibiotics are administered. However, the effects thereof are not sufficient. Also, such a medicament has an effect in the beginning, but the effect is sometimes reduced due to the acquisition of the drug resistance. Moreover, there are concerns that the medicaments may adversely affect the human body.

Accordingly, attempts to find measures which do not use medicaments such as antibiotics have been made, and as an example thereof, use of the bioactivities of natural components extracted from plants is focused on. Of the components, polyphenols such as anthocyanins contained in the fruit of blackcurrant are known to have an antiviral action (an action of deactivating viruses) against influenza virus and use thereof for preventing or treating diseases has been proposed.

For example, patent document 1 discloses a feed capable of preventing or treating a disease of an aquatic organism due to the antiviral action, the antibacterial action of blackcurrant fruit and a fish farming method using the feed, which do not use medicaments such as antibiotics. Patent document 1 describes that the feed and the method can increase the survival rate of an aquatic organism and also improve the quality of an edible aquatic organism.

As means for promoting the growth of an organism or increasing the body weight, it has been found that methionine, which is an essential amino acid that animals cannot synthesize in the body, contributes to the promotion of the growth of an organism, and attempts to add methionine to a feed have been made.

Patent document 1 describes that blackcurrant fruit is believed to have an effect of promoting the growth of an organism. It is speculated that this is because blackcurrant fruit contains growth promoting substances such as amino acids including methionine.

However, the inventors of the present application have made the following findings. Blackcurrant fruit contains components which have a fat burning action such as anthocyanins. Thus, the increase in the body weight of the subject organism achieved by the growth promoting effect of blackcurrant fruit is canceled out to some extent by the fat burning action when the feed described in patent document 1 is used, and the effect is insufficient when the main purpose is to increase the body weight.

Moreover, at present, methionine is produced from a petroleum-derived material through complicated steps, and the mass production requires an adequate facility. Thus, the environmental burden is relatively large, and it is doubtful whether the demand can be met in the future.

Therefore, a more effective new solution to the food problem, which is expected to become more serious in the near future, is required.

Prior Art Documents

Patent Document

Patent Document 1: JP-A-2016-13122

Summary of the Invention

Problems that the Invention is to Solve

In view of the above points, an object of the invention is to provide a feed which uses blackcurrant pomace as a raw material and which has a stronger effect of increasing the body weight of the subject organism, a production method thereof and a raising method using the feed.

Means for Solving the Problems

In order to solve the problems, the feed according to the invention includes fermented blackcurrant fruit.

The feed of the invention includes 0.01 to 2 mass% of the fermented blackcurrant fruit in terms of the dry mass.

The blackcurrant fruit is pomace of blackcurrant fruit and contains at least the pericarp.

The fermented blackcurrant fruit may have been fermented by at least one species selected from the group consisting of species of *Aspergillus, Bifidobacterium, Penicillium, Chrysosporium, Trichoderma, Acremonium, Candida, Mucor, Rhizopus, Monascus* and *Absidia.*

The method for raising an organism of the invention is a method in which an organism to be raised is caused to take the feed of the invention at least once a week.

The method for producing a feed of the invention is a method for producing the feed of the invention by a production method including fermenting blackcurrant fruit using at least one species selected from the group consisting of species of *Aspergillus, Bifidobacterium, Penicillium, Chrysosporium, Trichoderma, Acremonium, Candida, Mucor, Rhizopus, Monascus* and *Absidia.*

The production method may be a production method including a step of producing a fermentation starter derived from the blackcurrant fruit by seeding an *Aspergillus* species to the blackcurrant fruit, a step of obtaining fermented blackcurrant fruit by adding water to the fermentation starter derived from the blackcurrant fruit and heating the fermentation starter and a step of adding the fermented blackcurrant fruit to a feed.

Effects of the Invention

With the feed of the invention, a significant effect of increasing the body weight of an organism to be raised is obtained due to a growth promoting substance that the fermented product of blackcurrant fruit (including a processed product thereof) contains. Moreover, a preventive and therapeutic effect on a disease of the subject organism is also expected due to the antiviral and antibacterial actions of the fermented product.

According to the invention, a stronger effect of increasing the body weight than that obtained by a feed containing unfermented blackcurrant is obtained. It is believed that this is because proteins contained in blackcurrant fruit are decomposed by fermentation, thereby increasing the amounts of amino acids such as methionine, and because a decrease of a component having a fat burning effect in blackcurrant fruit also contributes to the effect at the same time.

Moreover, the method for producing a feed of the invention does not depend on the petroleum resources and does not require a large-scale production facility. Thus, the feed of the invention can be obtained while the environmental burden is reduced. In particular, use of pomace of blackcurrant fruit which remains after removing juice can reduce the cost of the material and can also contribute to the environmental protection through the effective utilization of the resource and the reduction of the waste.

Mode for Carrying Out the Invention

Embodiments of the invention are explained in detail below.

The feed of the invention contains fermented blackcurrant fruit (sometimes referred to as "fermented blackcurrant" below).

The term "blackcurrant fruit" disclosed in the present specification includes both of the whole fruit (entire fruit) and a part of fruit (pericarp, flesh, seeds or the like). However, from the point that the ratios of polyphenols such as anthocyanins and proteins are high, the pericarp is preferable. Proteins are decomposed into amino acids such as methionine through fermentation and are believed to contribute to the effect of increasing the body weight. Besides, it has been confirmed that the amounts of anthocyanins, which are contained in the pericarp in large amounts, are reduced considerably through fermentation. It is highly likely that this is because anthocyanins are converted into a composite with an amino acid, another polyphenol from the free state, rather than disappearing. The composite obtained in this manner is also thought to contribute to the effect of increasing the body weight and the preventive and therapeutic effect on a disease, which the invention aims.

The pomace which remains after pressing for juice (water) mainly comprises the pericarp, and the pomace is used in the invention. The pomace may be dried once, but pomace which still contains water to some extent can also be used. Conventionally, only the blackcurrant juice has been used and the pomace which remains after pressing for juice (water) has been often discarded, but the pericarp is a used effectively in the invention. Thus, the production cost of the feed can be reduced, and the invention can also contribute to the environmental protection through the effective utilization of the resource and the reduction of the waste.

Moreover, the blackcurrant fruit may be processed product of blackcurrant fruit. The "processed product of blackcurrant fruit" means a product obtained by subjecting raw blackcurrant fruit to any physical or chemical treatment to such an extent that the object of the invention is not disturbed, such as a frozen product or a dried product.

As the processed product of blackcurrant fruit, commercially available products can also be used. An example of the commercially available products of blackcurrant pericarp is "blackcurrant pericarp" (product number S0001) manufactured by Sujon Japan Co. Ltd.

The blackcurrant fruit used in the invention may be either raw fruit or a processed product such as those described above. Therefore, when the term "blackcurrant fruit" is simply used in the specification, such a processed product is also included.

In the invention, fermented blackcurrant pomace obtained by fermenting the blackcurrant fruit described above is used.

The species used for the fermentation is not particularly limited. For example, a fermentation method using a species of *Aspergillus,* lactic acid bacteria, *Bifidobacterium, Bacillus subtilis* var *natto, Penicillium, Chrysosporium, Trichoderma, Acremonium, Candida, Mucor, Rhizopus, Monascus, Absidia* is used. One of the species may be used alone, or a combination of two or more kinds thereof may be used. The species used is preferably a species of *Aspergillus, Bifidobacterium* and *Rhizopus* from the viewpoint of safety.

The *Aspergillus* species are not particularly limited. Examples thereof include *Aspergillus oryzae, Aspergillus sojae, Aspergillus niger, Aspergillus tamarii, Aspergillus kawachii, Aspergillus awamori, Aspergillus saitoi* but *Aspergillus oryzae* or *Aspergillus kawachii,* which are safe microorganisms that have been long used for brewing soy sauce, soybean paste and which are used in the Japanese industry highly frequently, are preferable.

The lactic acid bacteria are not particularly limited. Examples thereof include those of *Lactobacillus, Lactococcus, Streptococcus, Enterococcus.*

The *Bifidobacterium* species are not particularly limited. Examples thereof include *Bifidobacterium bifidum, Bifidobacterium longum, Bifidobacterium longum* subspecies *infantis, Bifidobacterium breve, Bifidobacterium adolescentis*.

The *Rhizopus* species are not particularly limited. Examples thereof include *Rhizopus oligosporus.*

With respect to the conditions of the fermentation, preferable conditions vary with the species used, but fermentation may be conducted under conditions which are generally used for each species.

The method for fermenting the blackcurrant fruit is explained below referring to an example using an *Aspergillus* species. However, the fermentation method is not limited to the example, and the fermentation can be conducted by appropriately adjusting the temperature conditions in accordance with the following method when another species is used.

<Production Step of Fermentation Starter>

An *Aspergillus* species is seeded to heat sterilized blackcurrant to produce a fermentation starter ("Koji") derived from blackcurrant. The conditions of the treatment here are not particularly limited. However, the materials are generally held at 20 to 48°C for around 25 to 72 hours, and the materials are preferably held at 30°C to 37°C for around 42 to 46 hours. In the step, the species contained in the fermentation starter generates enzymes, and the decomposition and the conversion of the proteins and the carbohydrates contained in the blackcurrant begin. The resultant product in which the *Aspergillus* species has proliferated on the blackcurrant is called "a blackcurrant fermentation starter" in the specification.

<Step of Adding Water and Heating>

After adding water to the blackcurrant fermentation starter obtained in the above manner, the blackcurrant fermentation starter is heated at 30 to 65°C, preferably at about 50 to 55°C, and held for about 5 to 72 hours, preferably for about 12 to 48 hours to cause hydrolysis. Fermented blackcurrant can be thus obtained. By holding at an appropriate temperature in the step, the activities of the enzymes generated by the fermentation starter are enhanced, and the decomposition and the conversion of the proteins and the carbohydrates contained in the blackcurrant can be further advanced. The resultant product which is obtained by hydrolyzing the blackcurrant fermentation starter in this manner and which contains amino acids which are the decomposition products of the proteins and glucose which is the decomposition product of the carbohydrates is called "fermented blackcurrant" in the specification. In general, it is believed that the *Aspergillus* species dies at about 50°C or higher, but the *Aspergillus* species can also be kept alive in the fermented blackcurrant according to the need by lowering the treatment temperature or reducing the treatment period.

The fermented blackcurrant obtained is dried according to the need for storage or use. The drying conditions are not particularly limited and may be appropriately adjusted according to the need as described above. However, the fermented blackcurrant can be dried generally at around 40 to 80°C.

Considering the easiness in handling, the efficiency of absorption or the like, the fermented blackcurrant is preferably in the form of powder and is more preferably powder of fine particles. To obtain powder thereof, the fermented blackcurrant may be subjected to processing such as cutting or crushing. However, because the proteins and the carbohydrates in the fermented blackcurrant have been decomposed as described above, the fermented blackcurrant can be crushed easily by simply applying slight force after drying. The powder is preferably fine powder having a mean particle size of around 5 to 150 μm. The particle size of the fermented blackcurrant powder obtained can be measured, for instance, by the sieve analysis test in accordance with JIS Z8815.

The feed of the invention contains the fermented blackcurrant described above. The fermented blackcurrant can be used alone, but a certain amount of the fermented blackcurrant may be added to a raw material composition according to the conventional formulation which has been used for each organism and the resultant mixture may be formed into powder, pellets, a lump or another desired form.

The active ingredient content of the blackcurrant fruit varies with the place of origin of the blackcurrant fruit, the part used, the processed state. Thus, the amount in the case of a mixed feed is preferably adjusted in such a manner that the desired effects can be obtained, and the amount cannot be determined uniformly. As a standard in the case of the incorporation of fermented blackcurrant obtained from pomace of blackcurrant fruit having an average active ingredient content, the amount in terms of the dry mass is preferably 0.01 to 2 mass%, more preferably 0.1 to 1 mass%, particularly preferably 0.2 to 0.5 mass%. When the amount is 0.01 mass% or more, the effect of increasing the body weight, which the invention aims, are obtained easily. An amount exceeding 2 mass% does not necessarily cause problems. However, addition in an excessive amount does not have any advantage but sometimes disturbs the nutritional balance and may cause a problem of deteriorated taste when the subject organism is an edible organism or a problem in the production (granulation). In this regard, the amount in terms of the dry mass indicates the ratio obtained by converting the value into the mass containing substantially no water both in the case of the fermented blackcurrant and the case of other raw materials.

The production method of the feed is not particularly limited, and a method in which a raw material composition containing the fermented blackcurrant described above is supplied for example to a single-axis or multi-axis extruder and granulated into pellets of dry type or moist type according to a general method can be used.

Here, when pellets are produced, in general, the raw material composition of the feed is formed into granules by heat forming. The conditions of heating here are not particularly limited but are preferably at 100 to 120°C for 15 to 30 minutes to minimize the thermal decomposition of the components contained in the raw material composition. Moreover, when the raw material composition of the feed is mixed, fat or oil is preferably added to improve the formability. The kind of fat or oil is not particularly limited but is preferably fish oil or palm oil in view of the cost.

Although the raw material composition of the feed varies depending on the kind of subject organism, the form of the feed, oil cake, grains, fish oil, vitamins, minerals are generally added to proteins such as fish powder, meat powder or powdered skimmed milk.

Alternatively, a method in which the fermented blackcurrant is adhered to the surface of pellets obtained from a raw material composition without adding the fermented blackcurrant can also be used. When the fermented blackcurrant does not adhere easily because the surface of the pellets is flat and smooth or dry, fat or oil can be used as a binder to increase the amount of the adhered fermented blackcurrant. The fat or oil used is not particularly limited as in the above-described case, but fish oil or palm oil can be used. Also, it is of course possible to adhere the fermented blackcurrant to the surface of pellets obtained by adding the fermented blackcurrant according to the need.

The subjects to which the feed of the invention is used are organisms in general which are raised mainly for the purpose of food or for working, regardless of whether the organisms are terrestrial organisms or aquatic organisms, and include livestock such as cows, pigs, horses, sheep and camels, poultry such as chickens, ducks, crossbreeds between wild and domestic ducks, pheasants, quails, turkeys, guinea fowl, peafowl, geese, pigeons, ostriches and emus, various kinds of fish, crustaceans such as shrimps and crabs and shellfish as well as turtles, soft-shelled turtles.

Although it varies depending on the kind of the organism to be raised, in order to obtain the desired effect of increasing the body weight, the feed of the invention obtained in the above manner is preferably given to the subject organism continuously at a frequency of once a week or more for several days or longer and more preferably given to the subject organism continuously at a frequency of once a day or more for several days or longer.

The method for giving the feed is not particularly limited, and the feed of the invention can replace a conventionally given feed entirely or partially when the feed is used. Also, a conventional feed and the feed of the invention can be mixed and used.

The amount of the feed to be given is preferably adjusted appropriately depending on the kind of the subject organism, the growing state, the growing environment in such a manner that the active ingredient of the blackcurrant fruit is taken into the body of the subject organism to exert the desired effects.

By causing the organism to be raised to take the feed, effects of significantly increasing the body weight of the subject organism and preventing a disease can be obtained.

It has not been elucidated yet how these effects are obtained. However, as described above, it is speculated that an increase in the methionine and methionine derivative contents due to the fermentation of blackcurrant as compared with unfermented blackcurrant contributes to the increase in the body weight of the subject organism. It is also speculated that a decrease in the amounts of free polyphenols such as anthocyanins having a fat burning action due to the fermentation of blackcurrant also affects the effect. In this regard, however, the fat burning action of free anthocyanins may also be used according to the need, and for example, meat with a low fat content can be obtained by using a feed using a mixture of unfermented blackcurrant and the fermented blackcurrant.

Moreover, with respect to the prevention and the treatment of a disease of the subject organism, a composite of anthocyanins contained in the blackcurrant with another polyphenol or an amino acid generated by fermentation is believed to exert antiviral and antibacterial actions.

An example of the improvement of growth failure is the improvement of incidence of egg binding (also called a stuck egg, a bound egg or the like) in hens. Here, egg binding is a phenomenon in which a hen cannot lay an egg properly for various reasons during the production process of the egg in the oviduct and the egg is stuck. Egg binding is a major cause of death of hens. The mechanism for this effect is also unclear, but it is speculated that methionine contained in the fermented blackcurrant is involved.

Also, because the feed of the invention contains the fermented blackcurrant, an effect of inhibiting the growth of mold is also obtained. It is speculated that the effect is due to the antibacterial action of the fermented blackcurrant.

Furthermore, the feed of the invention is tolerant of temperature change and can maintain the active ingredient even when the feed of the invention is treated at a high temperature of 100 to 120°C during heat forming.

Examples

Next, Examples are explained together with Comparative Examples. However, the invention is not limited to the Examples. The contents below are based on the mass unless otherwise noted.

[Production Example (Production Example of Fermented Blackcurrant) 1]

Raw blackcurrant fruit was pressed using a press to squeeze out water, and blackcurrant pomace was obtained. An *Aspergillus* species (*Aspergillus oryzae*) was seeded to the blackcurrant pomace obtained and held at 30°C to 37°C for 46 hours to prepare a blackcurrant fermentation starter. After adding 3.8 kg of water to 10 kg of the blackcurrant fermentation starter obtained, the blackcurrant fermentation starter was held at 50 to 55°C for about 24 hours and to be hydrolyzed, and fermented blackcurrant was thus obtained.

The fermented blackcurrant obtained was held at 55°C for 24 hours to be dried and crushed with a jet mill, and fermented blackcurrant powder was thus obtained.

<Measurement of Anthocyanin Content>

The free anthocyanin contents of following samples were examined by liquid chromatography; the raw material blackcurrant used in the Production Example (sample a), the blackcurrant fermentation starter obtained in the Production Example by adding the *Aspergillus* species to the raw material (sample b), the hydrolysate of the blackcurrant fermentation starter (sample c) and the fermented blackcurrant powder obtained by drying and crushing the hydrolysate (sample d). The results are as follows.

Results Sample a: 0.479%
Sample b: 0.079%
Sample c: 0.005%
Sample d: 0.004%

As shown by the above results, it was confirmed that the free anthocyanin content after the hydrolysis was about one hundredth of that of the raw material.

[Production Example (Production Example of Fermented Blackcurrant) 2]

To examine the change in the antioxidative action of blackcurrant by fermentation, blackcurrant fermentation starters and fermented blackcurrant were obtained by the same methods as in Production Example 1 of the fermented blackcurrant except that the species used for the fermentation was changed from *Aspergillus oryzae* to *Aspergillus kawachii* or *Aspergillus awamori*.

<Antioxidative Test>

Unfermented blackcurrant pomace was named sample A; the blackcurrant fermentation starter obtained using *Aspergillus kawachii* was named sample B; the blackcurrant fermentation starter obtained using *Aspergillus awamori* was named sample B'; the fermented blackcurrant obtained using *Aspergillus kawachii* was named sample C; and the fermented blackcurrant obtained using *Aspergillus awamori* was named sample C'.

To 1 g of each of the five samples, 10 ml of an aqueous 1% (v/v) trifluoroacetic acid solution was added, and extraction was conducted in a dark place at room temperature for 16 hours, and an extract was obtained. The extract obtained was centrifuged (3000 rpm, 20 minutes, 4°C) to obtain a supernatant liquid. After adding 10 ml of an aqueous 1% (v/v) trifluoroacetic acid solution to the residue and stirring the mixture, the mixture was centrifuged again, and the supernatant liquid obtained was added to the supernatant liquid obtained first.

The supernatant liquids of the samples were respectively diluted twofold, put into a syringe (Terumo syringe) and filtered through the membrane filter (LH-0.45 $\mu$M) attached to the tip of the syringe. The filtrates were used as the sample for the measurement.

The changes of fluorescence intensity of the samples were measured with a microplate reader based on the H-ORAC analysis method to calculate the H-ORAC values per 1 g sample ($\mu$molTE/g). The results are as follows.

Results Sample A : 117.02 $\mu$molTE/g
Sample B : 129.15 $\mu$molTE/g
Sample B' : 101.93 $\mu$molTE/g
Sample C : 91.42 $\mu$molTE/g
Sample C' : 63.39 $\mu$molTE/g

As shown by the above results, the antioxidative actions of the fermented blackcurrant samples (samples C and C') were lower than that of the unfermented blackcurrant (sample A). However, from the results of the measurement of the anthocyanin contents, the anthocyanin content after the hydrolysis was about a hundredth of that of the raw material. A decrease in the antioxidative action to the extent that was expected from the results was not observed. Therefore, it is speculated that the anthocyanins contained in the blackcurrant did not disappear due to the fermentation but were converted into a composite with another polyphenol or an amino acid.

[Example 1 and Comparative Example 1]

Feeds were prepared using the fermented blackcurrant obtained in Production Example 1 as follows, and testing using chickens was conducted.

That is, commercially available mixed feeds for chickens which did not contain blackcurrant or fermented blackcurrant were used as the feeds of Comparative Example 1.

The fermented blackcurrant obtained in Production Example 1 was added to the feeds of Comparative Example 1 at a ratio of 0.2 mass%, and the feeds were mixed well to adhere the fermented blackcurrant evenly to the surface of the mixed feeds. The feeds of Example 1 were thus prepared.

<Evaluation Method>

The feeds of Example 1 and Comparative Example 1 were evaluated by the average body weights of the chickens and the feed efficiencies. The specific methods are as follows. The results of the average body weights and the feed efficiencies obtained are shown in Table 1.

1. Average Body Weight:

One hundred chickens as the subject organism were divided into two groups (each including 50 chickens), namely a group to which the feeds of Example 1 were given and a group to which the feeds of Comparative Example 1 were given. A same amount of feed was given to the groups once a day when chickens were 1 to 49 days old. Ten chickens were extracted at random from the groups respectively as the intervention group and the control group, and the average body weights of the respective groups were measured and recorded for 49 days.

2. Feed Efficiency:

A same amount of feed was given to the groups every day, and the remaining amounts were measured on the following days to record the amounts of feed that the chickens ate on the previous days. The total feed amounts, which are the total amounts of feed consumed in the 49 days, were determined. The total feed amounts were divided by the respective average body weights as in the following equation to determine the feed efficiencies. The smaller the feed efficiency is, the stronger the effect of increasing the body weight is.

$$\text{Feed Efficiency} = \text{Total Feed Amount (g)}/\text{Average Body Weight (g)}$$

[Table 1]

| Day old | Average Body Weight (g) | | Total Feed Amount (g) | | Feed Efficiency | |
| --- | --- | --- | --- | --- | --- | --- |
| | Comparative Example 1 | Example 1 | Comparative Example 1 | Example 1 | Comparative Example 1 | Example 1 |
| 1 | 50 | 50 | 32 | 33 | - | - |
| 7 | 88 | 113 | 205 | 193 | 2.33 | 1.71 |
| 14 | 202 | 291 | 419 | 521 | 2.07 | 1.79 |
| 21 | 429 | 583 | 907 | 1110 | 2.11 | 1.90 |
| 28 | 812 | 988 | 1704 | 2171 | 2.10 | 2.20 |
| 35 | 1388 | 1672 | 2839 | 3319 | 2.05 | 1.99 |
| 42 | 2075 | 2336 | 4034 | 4476 | 1.94 | 1.91 |
| 49 | 2644 | 3021 | 5347 | 5911 | 2.02 | 1.96 |

The results in Table 1 show that the chickens of the group to which the feeds of Example 1 containing the fermented blackcurrant were given grew faster than the chickens of the group to which the feeds of Comparative Example 1 were given, and in particular a significant difference was observed in the feed efficiency at 7 days of age between Example 1 and Comparative Example 1. In this regard, the standard raising period (days) of broilers is recently around 35 days.

[Example 2 and Comparative Example 2]

A feed for fish farming was prepared using the fermented blackcurrant obtained in Production Example 1 as follows, and testing using yellowtail was conducted.

That is, 0.2 mass% of the fermented blackcurrant powder obtained in Production Example 1 and 0.5% of fish oil were added to commercially available fish feed 1 in the crumble form and fish feed 2 in the pellet form which did not contain blackcurrant or fermented blackcurrant, and the feed was mixed well to adhere the fermented blackcurrant powder evenly to the surface of the feed. The feed of Example 2 was thus obtained.

The feed of Comparative Example 2 was prepared in the same manner as in Example 2 except that unfermented blackcurrant powder was used instead of the fermented blackcurrant powder of Production Example 1 added in Example 2.

<Evaluation Method>

About 15000 yellowtail fish as the subject organism were divided into two groups, namely a group to which the feed of Example 2 was given and a group to which the feed of Comparative Example 2 was given. A same amount of feed was given to the groups three times a week for about five months, and the average body weights, the conversion coefficients over the period and the conversion cost were determined by the following methods.

1. Average Body Weight (kg)

Ten fish each were extracted at random from the groups respectively as the intervention group and the control group to determine the average body weights, and the average body weights of the respective groups were recorded for about five months.

2. Conversion Coefficient Over Period

From the total amount of feed given and the increase in the average body weight determined above, the conversion coefficient was determined by the following equation. The smaller the conversion coefficient over the period is, the stronger effect of increasing the body weight the feed has.

Conversion Coefficient Over Period

$$= \text{Total Feed Amount (kg)/Increase in Average Body Weight (kg)}$$

3. Conversion Cost (Yen)

From the unit price of the feed used and the conversion coefficient over the period determined above, the conversion cost was determined by the following equation. In this regard, the values were calculated regarding the unit price of the feed containing the unfermented blackcurrant powder as 220 yen and regarding the unit price of the feed containing the fermented blackcurrant powder as 225 yen.

Conversion Cost (Yen)

$$= \text{Conversion Coefficient Over Period} \times \text{Unit Price of Feed (Yen)}$$

[Table 2]

| | Average Body Weight (kg) | | Conversion Coefficient Over Period | | Conversion Cost (Yen) | |
|---|---|---|---|---|---|---|
| | Example 2 | Comparative Example 2 | Example 2 | Comparative Example 2 | Example 2 | Comparative Example 2 |
| At the start of test | 1.13 | 1.09 | - | - | - | - |
| One month later | 1.52 | 1.50 | 2.01 | 2.01 | 452 | 443 |
| Three months later | 1.74 | 1.72 | 2.24 | 2.31 | 503 | 507 |
| Five months later | 1.98 | 1.91 | 2.18 | 2.38 | 490 | 524 |

As shown in Table 2, it was confirmed that the group to which the feed containing the fermented blackcurrant of Example 2 was given had a smaller conversion coefficient over the period than the group to which the feed containing the unfermented blackcurrant of Comparative Example 2 was given and that the feed of Example 2 had a superior effect of increasing the body weight. It was also confirmed that Example 2 had a lower conversion cost than Comparative Example 2 and could raise the yellowtail at a lower cost.

Industrial Applicability

The feed of the invention can be used for organisms in general including livestock such as cows, pigs, horses, sheep and camels, poultry such as chickens, ducks, crossbreeds between wild and domestic ducks, pheasants, quails, turkeys, guinea fowl, peafowl, geese, pigeons, ostriches and emus and aquatic organisms such as fish and shellfish. In particular, the invention is of high utility value for raising chickens such as broilers and Shamo chickens, which are desired to grow in a short period.

**Claims**

**1.**    A feed comprising fermented blackcurrant fruit comprising 0.01 to 2 mass% of the fermented blackcurrant fruit in terms of the dry mass, wherein the blackcurrant fruit is pomace of blackcurrant fruit and contains at least a pericarp.

2. The feed according to claim 1, wherein the fermented blackcurrant fruit has been fermented by at least one species selected from the group consisting of species of *Aspergillus, Bifidobacterium, Penicillium, Chrysosporium, Trichoderma, Acremonium, Candida, Mucor, Rhizopus, Monascus* and *Absidia.*

3. A method for raising an organism comprising causing an organism to be raised to take the feed according to claim 1 or 2 at least once a week.

4. A method for producing the feed according to claim 1 or 2, comprising
fermenting blackcurrant fruit using at least one species selected from the group consisting of species of *Aspergillus, Bifidobacterium, Penicillium, Chrysosporium, Trichoderma, Acremonium, Candida, Mucor, Rhizopus, Monascus* and *Absidia.*

5. The method for producing the feed according to claim 4, comprising

a step of producing a fermentation starter derived from the blackcurrant fruit by seeding an *Aspergillus* species to the blackcurrant fruit,
a step of obtaining fermented blackcurrant fruit by adding water to the fermentation starter derived from the blackcurrant fruit and heating the fermentation starter, and
a step of adding the fermented blackcurrant fruit to a feed.

**Patentansprüche**

1. Futtermittel, das fermentierte schwarze Johannisbeerfrüchte aufweist, die 0,01 bis 2 Massenprozent der fermentierten schwarzen Johannisbeerfrüchte, bezogen auf die Trockenmasse, aufweisen, wobei die schwarzen Johannisbeerfrüchte ein Trester von schwarzen Johannisbeerfrüchten sind und mindestens ein Perikarp enthalten.

2. Futtermittel nach Anspruch 1, wobei die fermentierten schwarzen Johannisbeerfrüchte durch mindestens eine Art fermentiert worden sind, die aus der Gruppe ausgewählt worden ist, die Arten von *Aspergillus, Bifidobacterium, Penicillium, Chrysosporium, Trichoderma, Acremonium, Candida, Mucor, Rhizopus, Monascus* und *Absidia* umfasst.

3. Verfahren zur Aufzucht eines Organismus, bei dem ein aufzuziehender Organismus veranlasst wird, mindestens einmal wöchentlich das Futtermittel nach Anspruch 1 oder 2 aufzunehmen.

4. Verfahren zur Herstellung des Futtermittels nach Anspruch 1 oder 2, welches umfasst:
ein Fermentieren von schwarzen Johannisbeerfrüchten unter Verwendung mindestens einer Art, die aus der Gruppe ausgewählt worden ist, die Arten von *Aspergillus, Bifidobacterium, Penicillium, Chrysosporium, Trichoderma, Acremonium, Candida, Mucor, Rhizopus, Monascus* und *Absidia* umfasst.

5. Verfahren zur Herstellung des Futtermittels nach Anspruch 4, welches umfasst:

einen Schritt der Herstellung eines aus der schwarzen Johannisbeerfrüchten gewonnenen Fermentationsstarters, indem die schwarze Johannisbeerfrüchten mit einer Art von *Aspergillus* geimpft werden,
einen Schritt der Gewinnung fermentierter schwarzer Johannisbeerfrüchten durch ein Hinzufügen von Wasser an den aus den schwarzen Johannisbeerfrüchten gewonnenen Fermentationsstarter und ein Erhitzen des Fermentationsstarters, und
einen Schritt der Zugabe der fermentierten schwarzen Johannisbeerfrüchte zu einem Futtermittel.

**Revendications**

1. Aliment pour animaux comprenant un produit fermenté de fruits de cassis comprenant 0,01 à 2 % en masse du produit fermenté de fruits de cassis en termes de masse sèche, dans lequel le produit de fruits de cassis est un marc de fruits de cassis et contient au moins un péricarpe.

2. Aliment pour animaux selon la revendication 1, dans lequel le produit fermenté de fruits de cassis a été fermenté par au moins une espèce choisie dans le groupe constitué d'espèces *d'Aspergillus, Bifidobacterium, Penicillium, Chrysosporium, Trichoderma, Acremonium, Candida, Mucor, Rhizopus, Monascus* et *Absidia.*

3. Procédé d'élevage d'un organisme comprenant le fait d'amener un organisme à être élevé pour prendre l'aliment pour animaux selon la revendication 1 ou 2 au moins une fois par semaine.

4. Procédé de production de l'aliment pour animaux selon la revendication 1 ou 2, comprenant
la fermentation de fruits de cassis en utilisant au moins une espèce choisie dans le groupe constitué d'espèces *d'Aspergillus, Bifidobacterium, Penicillium, Chrysosporium, Trichoderma, Acremonium, Candida, Mucor, Rhizopus, Monascus* et *Absidia.*

5. Procédé de production de l'aliment pour animaux selon la revendication 4, comprenant

une étape de production d'un démarreur de fermentation dérivé du produit de fruits de cassis par ensemencement d'une espèce *d'Aspergillus* dans le produit de fruits de cassis,
une étape d'obtention de produit fermenté de fruits de cassis par ajout d'eau au démarreur de fermentation dérivé du produit de fruits de cassis et chauffage du démarreur de fermentation, et
une étape d'ajout du produit fermenté de fruits de cassis à un aliment pour animaux.

**EP 3 536 162 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016013122 A